Europäisches Patentamt

European Patent Office

Office européen des brevets

(19)

(11) Publication number: **0 352 328**

**A1**

(12)

# EUROPEAN PATENT APPLICATION
## published in accordance with Art. 158(3) EPC

(21) Application number: 88908990.0

(22) Date of filing: 17.10.88

(86) International application number:
PCT/JP88/01053

(87) International publication number:
WO 89/04327 (18.05.89 89/11)

(51) Int. Cl.⁴: **C07K 15/04 , C12N 5/00 , C12N 15/00 , C12P 21/00 , //A61K39/395,G01N33/577, (C12P21/00,C12R1:91)**

(30) Priority: 09.11.87 JP 280883/87

(43) Date of publication of application:
31.01.90 Bulletin 90/05

(84) Designated Contracting States:
AT BE CH DE FR GB IT LI NL SE

(71) Applicant: **TEIJIN LIMITED**
**6-7, Minamihonmachi 1-chome Chuo-ku**
**Osaka-shi Osaka 541(JP)**

(72) Inventor: **SAWADA, Shuzo**
**Teijin Shataku F 5-20-2, Tamadaira**
**Hino-shi Tokyo 191(JP)**
Inventor: **MASUHO, Yasuhiko**
**Teijin Shataku C 5-20-2, Tamadaira**
**Hino-shi Tokyo 191(JP)**
Inventor: **KAWAMURA, Takashi**
**855 East River Road,**
**Tucson, AZ 85718(US)**
Inventor: **SASAKI, Satoshi**
**Haimu SI 1-6-14, Shinmei**
**Hino-shi Tokyo 191(JP)**

(74) Representative: **Cookson, Barbara Elizabeth et al**
**WITHERS & ROGERS 4 Dyer's Buildings**
**Holborn**
**London EC1N 2JT(GB)**

(54) **HUMAN MONOCLONAL ANTIBODY AGAINST ASPERGILLI.**

(57) Monoclonal antibody against aspergilli; hybridoma formed by cell fusion of human lymphocyte with lymphoid established cells, and cell lines of its origin; transformed cells obtained by transforming human lymphocyte with EB virus; and process for producing human monoclonal antibody against aspergilli, which comprises culturing the cells and collecting human monoclonal antibody from the cultured product, are disclosed. The monoclonal antibody is expected to be useful for diagnosis and treatment of aspergillosis.

- 1 -

<u>DESCRIPTION</u>

TITLE OF THE INVENTION

Human Monoclonal Antibodies to Aspergillus

TECHNICAL FIELD

The present invention relates to human monoclonal antibodies to <u>Aspergillus</u> and hybridoma producing the same, and a process for production of those monoclonal antibodies. Those monoclonal antibodies are useful for the diagnosis and treatment of aspergillosis caused by <u>Aspergillus</u> infection.

BACKGROUND ART

<u>Aspergillus</u> is present in the soil and air, and is one of the most commonly found fungi. The genus <u>Aspergillus</u> includes more than 150 species, such as <u>A. fumigatus</u>, <u>A. flavus</u>, <u>A. nidulans</u>, <u>A. niger</u>, <u>A. terreus</u>, but the most important species as a pathogen of aspergillosis, etc. is <u>A. fumigatus</u>. A mycelium of <u>Aspergillus</u> differs in the form of separate hypha, and the conidiophore elongates in air. Further a top of the conidiophore has a vesicle expanding to form a subsphere, and the vesicle has flask-shaped phialides on its surface, whereas conidia are produced.

Aspergillosis is a kind of diseases caused by an infection of <u>Aspergillus</u>, and a main pathogen thereof is <u>A. fumigatus</u>. Among the aspergillosis, the disease most frequently encountered is pulmonary aspergilloma wherein <u>A. fumigatus</u> invades and grows in a cavity formed due to pulmonary tuberculosis. Moreover, a immunocompromised patient such as a leukemia patient, an organ-transplant recipient has a high risk of infection with <u>Aspergillus</u>, which causes pneumonia. In addition, a pathogen of allergic bronchopulmonary aspergillosis, disseminated aspergillosis, otomycosis, and keratomycosis is <u>Aspergillus</u>.

Generally, deep seated mycosis including aspergillosis have rapidly increased, due to the widespread use of antibiotics, antitumor agents, and steroid hormones. Mycosis observed in autopsy include candiasis, aspergillosis and cryptococcosis in a ratio of 30%, 20% and 10%.

Amphotericin B, Flucytosine, Miconazole, and Ketoconazole are used for the therapy of deep seated mycosis including aspergillosis. In the case of aspergillosis, a combination of Amphotericin B and Flucytosine is mainly used but Amphotericin B causes severe side effects such as renal toxicity, anemia, and gastrointestinal disorders, and although Flucytosine has a relatively lower toxicity, it is disadvantageous in that it causes side effects, such as a decrease of leucocytes and gastrointestinal disorders, and resistant microbes are readily developed. Therefore, as seen from the above, the current therapy is not satisfactory.

Deep seated mycosis mainly develops in immunocompromised patients such as patients of leukemia, cancer, acquired immunodeficiency syndrome (AIDS), organ transplantation, and autoimmune for which an immunosuppressant is administered, and the main pathogens are Candida, Aspergillus and Cryptococcus. To detect these fungi, there are a method wherein an antibody to these fungi present in a serum of a patient infected with these fungi is detected, and a method wherein an antigen of these fungi is detected. To detect an antibody to Aspergillus, a latex agglutination reaction, complement binding reaction, double diffusion in agar gel, and intradermal reaction, using as an antigen a culture supernatant of A. fumigatus or an extract thereof, are used. In the case of a patient in which the immune response is maintained, these methods can be used for diagnosis. But in the case of invasive pulmonary aspergillosis, which frequently develops in a patient not exhibiting an immune response, an antibody is difficult to produce, resulting in difficulties in diagnosis. Accordingly, a method wherein a fungal antigen is immunologically detected using an antibody to infective fungus is used. Currently, an antiserum obtained from an animal immunized with the fungus is used for this purpose, but a highly sensitive method using an antiserum has not been established to date.

In 1975, Milistein and Kohler first prepared a mouse monoclonal antibody. A monoclonal antibody, in comparison with a conventional antiserum, is advantageous in that (1) it exhibits high sensitivity, (2) it exhibits a high antibody titer, (3) it is highly reproducible, and (4) it can be obtained in a large amount by growing cells producing the monoclonal antibody (hybridoma). Many monoclonal antibodies to biological materials have been produced to date, and are used for therapy. For a therapy of fungi, a mouse monoclonal antibody to Candida is disclosed in, for example, Japanese Unexamined Patent Publication (KOKAI) No. 62-62000, but there is no report of a monoclonal antibody to Aspergillus. If a monoclonal antibody to Aspergillus is obtained, it would be useful for a diagnosis of aspergillosis, and if a human monoclonal antibody is obtained, it would probably become an effective therapeutic agent against Aspergillus, and would have very low side effects. Namely, as a therapeutic agent, a human monoclonal antibody is needed, and a mouse monoclonal antibody is not preferable. This is because, when a mouse monoclonal antibody is administered to a patient, an immune response occurs in the patient and antibodies to the heterologous mouse protein are produced, which antibodies not only inhibit the mouse monoclonal antibody but also cause a side effect due to the formed immune complex. Accordingly, a human monoclonal antibody is necessary for this therapy.

Namely, although both the mouse and human monoclonal antibodies are useful for diagnosis, the development of a human monoclonal antibody is desired for therapy.

Generally, a monoclonal antibody of human origin is produced by hybridoma obtained by cell fusion between a mouse myeloma cell, human myeloma cell or other established lymphoid cell and a human lymphocyte. Alternatively, it is produced by lymphoblastoid cells transformed with EB virus. Although from 1980 onward the production of many human monoclonal antibodies has been attempted, each process has characteristic problems. The production of a monoclonal antibody by hybridoma obtained from cell fusion between mouse myeloma cell and human lymphocyte is unstable, and the efficiency of a cell fusion between human myeloma cell and human lymphocyte is very low. Moreover, the production of a monoclonal antibody by lymphoblastoid obtained using an EB virus has a low yield and is unstable. In addition, since the EB virus can generate tumors, problems arise from the point of view of safety.

As described above, there are large obstacles to the establishment of a human monoclonal antibody-producing cell. Moreover, there is a problem of how to obtain a human lymphocyte immunized against _Aspergillus_. Human lymphocytes can be obtained from peripheral blood; a surgically removed spleen, lymph nodes or tonsils; or from bone marrow. Generally, although peripheral blood is readily available, the availability of other tissues is very limited. Moreover, in general, peripheral blood contains a small amount of B lymphocytes, and therefore, a very small amount of B lymphocytes activated by an antigen, and a large portion of such cells gather in the lymphatic tissues. Accordingly, it is difficult to obtain _Aspergillus_-specific B cells from peripheral blood. Even where the solid lymphoid tissues are used, the availability of a lymphocyte-donor sufficiently immunized against _Aspergillus_ is not high. Accordingly, due to the above-mentioned obstacles, human monoclonal antibodies to _Aspergillus_ have never been produced.

DISCLOSURE OF THE INVENTION

Accordingly, the present invention provides human monoclonal antibodies to _Aspergillus_.

The present invention also provides hybridoma formed by a cell fusion between a human lymphocyte and an established lymphoid cell and producing a monoclonal antibody to _Aspergillus_, and cell lines derived therefrom.

Moreover, the present invention provides a process for the production of a human monoclonal antibody to _Aspergillus_, characterized by culturing hybridoma formed by a cell fusion between a human lymphocyte and an established lymphoid cell and producing a monoclonal antibody to _Aspergillus_ or a cell line derived therefrom, and recovering the human monoclonal antibody to _Aspergillus_ from the cultured product.

Further, the present invention provides a transformed cell obtained by transforming a human lymphocyte with an EB virus and producing a human monoclonal antibody to _Aspergillus_, and cell line derived therefrom.

The present invention also provides a process for the production of a human monoclonal antibody to _aspergillus_, characterized by culturing a transformed cell obtained by transforming a human lymphocyte with an EB virus and producing a human monoclonal antibody to _Aspergillus_ or a cell line derived therefrom, and recovering the human monoclonal antibody from the cultured product.

Best Mode of Carrying Out the Invention

According to the present invention, first a human B-lymphocyte capable of producing an antibody to _Aspergillus_ is obtained, and, for example, by one of the following two procedures, a cell line producing a human anti-_Aspergillus_ monoclonal antibody is established. According to the first procedure, a human B-lymphocyte capable of producing a monoclonal antibody to _Aspergillus_ is, optionally after stimulation with a B-lymphocyte activating factor, fused with a myeloma cell (cell fusion partner). After about 3 to 5 weeks from the fusion, supernatants of developed hybridomas are sampled and tested for the presence of an antibody to _Aspergillus_. Hybridomas producing a specific antibody are then cloned, and clones exhibiting an excellent antibody producing ability are selected.

According to the second procedure, the above-mentioned lymphocyte derived from the tonsil is infected with the EB virus to transform the lymphocyte. Where a tonsil cell having a high ability to produce an antibody to _Aspergillus_ is used, although clones producing a _Aspergillus_-specific antibody are efficiently developed, since a major portion thereof is unstable in antibody production, they must be cloned at a precise timing with the addition of feeder cells. The cells thus obtained, producing a human anti-_Aspergillus_ monoclonal antibody, are cultured, and a supernatant thereof is collected which is then subjected to column chromatography or the like to purify the human monoclonal antibody.

Lymphocytes used in the present invention are contained in the human spleen, lymph node, tonsils, adenoids, bone marrow, and peripheral blood. Although lymphocytes from any source are used for the present invention, preferably, these lymphocytes are derived from a solid lymphoid tissue such as the tonsil, adenoid, spleen, bone marrow, or lymph node. Most preferably, a source is used which provides a lymphocyte having an ability to produce a large amount of antibodies to _Aspergillus_ when cultured in vitro with a lymphocyte activating factor and/or a _Aspergillus_ antigen.

For example, first, many human tonsils removed from patients suffering from tonsilitis are collected, and lymphocytes are separated from the collected tonsils.

The immune response to _Aspergillus_ varies remarkably, depending on the tonsil donors, and accordingly, lymphocyte sources are screened for a high ability to produce in vitro an antibody to _Aspergillus_. In this manner, a lymphocyte source having the highest ability to produce an antibody to _Aspergillus_ is obtained.

Where lymphocytes obtained from the thus selected lymphocyte source are used to construct hybridoma, preferably prior to the cell fusion, the lymphocytes are stimulated with a B-lymphocyte activating factor and/or an _Aspergillus_ antigen. The B-lymphocyte activating factor may be any substance which promotes the growth of B-lymphocyte, and includes, for example, pokeweed mitogen (PWM), B-cell growth factors (BCGF), protein A, phytohaemaglutinin (PHA) and concanavalin A. Preferably, 2 to 200 $\mu$g/ml PWM or one hundredth to one third volume of BCGF is used. As the _Aspergillus_ antigen, _Aspergillus_ cells per se or an antigen extracted from _Aspergillus_ cells is used. The cells are preferably used in an amount of $1 \times 10^5$ to $1 \times 10^8$ cells/ml. Although the method and condition of stimulation are not critical, for example, one hundredth to one third volume of BCGF and $1 \times 10^5$ to $1 \times 10^7$/ml lymphocytes are mixed, and cultured. The culturing temperature is 35°C to 40°C, and the culturing time is 3 to 10 days, preferably 4 to 6 days. Although any culture medium containing serum of human, bovine or horse origin may be used, media, such as RPMI 1640, containing fetal calf serum (FCS) are particularly preferable.

Myeloma cells used in the present invention are human myeloma cells, human B-lymphoblastoid cells, human B-lymphoma cells, mouse myeloma cells, hetero-myeloma cells derived from hybridoma of human lymphocyte and mouse myeloma cell, or the like. As mouse myeloma cell lines exhibiting an especially high fusion efficiency, P3 x 63Ag 8; P3-NS1/1-Ag4-1; P3 x 63Ag8U1; SP2/OAg 14;

P3 x 62Ag 8.653; MPC11-45.6, TG1.7; SP-1, and the like are preferable, although fundamentally any myeloma cell line can be used in the present invention.

The fusion between human lymphocyte and myeloma cell is carried out as follows. For example, antibody-producing cells and myeloma cells are mixed at a ratio of 10:1 to 1:10, preferably 1:1 to 1:3, to the mixture is added an appropriate cell fusion medium such as RPMI 1640 containing about 35% polyethylene glycol (approximate molecular weight 1,000 to 6,000) and about 7.5% dimethylsulfoxide, the mixture is stirred at a temperature between a room temperature and 37°C for 1 to several minutes, gradually diluted with RPMI 1640 supplemented with 10% FCS, and the cell concentration is adjusted to 1 to 5 x 10$^5$ cells/ml by a HAT (hypoxanthine-aminopterin-thymidine) selection medium. Then 0.2 ml of the mixture is distributed, for example, to each well of a 96-well plate, and cultured in air containing 5% $CO_2$, at 35°C to 38°C for 2 to 3 weeks. In the HAT medium, only hybridoma survives, and myeloma cells which are resistant to 8-azaguanine and myeloma X myeloma fused cells cannot survive (non-fused antibody-producing cells die in a few days).

After culturing, the antibody titer of the culture fluids is checked, to select and isolate hybridomas producing a desired antibody (cloning). This check of the antibody titer in a culture fluid can be carried out by a radioimmunoassay (RIA), Enzyme-linked immunosorbent assay (ELISA), or cell agglutination technique. The hybridoma producing the human anti-Aspergillus monoclonal antibody of the present invention, selected by the cloning, can be frozen and stored. Such a hybridoma cell line and/or cell line derived therefrom is cultured on a large scale to obtain a desired human monoclonal antibody from the culture supernatant. Alternatively, hybridoma can be transplanted to an animal to form a tumor, and a monoclonal antibody can be obtained from ascites or serum of the animal.

Where transformed cells are obtained by transformation with the EB virus, lymphocytes obtained from a source selected for the production of anti-Aspergillus antibody as described above are transformed with the EB virus. This method is generally known (for example, Steinitz et al., Nature 269, 420, 1977), and any EB virus may be used. For example, B95-8 cells are cultured in an RPMI medium supplemented with 10% FCS, and the culture supernatant is divided among vials which are then stored at -80°C, for later use as an EB virus source. When to be used, the virus sample is first thawed and then infected to $1 \times 10^7$ lymphocytes at a ratio of m.o.i. 0.01 to 1.0. The infection is carried out by incubation at 35 to 38°C for one hour. Subsequently, the lymphocytes are centrifuged, and infected lymphocytes are cultured at 37°C. As the medium, for example, RPMI1640 supplemented with 20% FCS is used, preferably with an addition of feeder cells such as mouse peritoneal cells. At a time at which the lymphoblast cells are fully grown, the culture supernatant is removed and tested for the presence of an antibody activity to Aspergillus. Lymphoblastoid cells producing the desired activity are selected and are cloned in a soft agar or by limiting dilution to obtain a subclone stably producing an antibody. To improve an antibody productivity of the lymphoblast cells thus obtained, the lymphoblastoid cell can be fused with myeloma cell to obtain hybridoma producing an anti-Aspergillus monoclonal antibody.

Human anti-Aspergillus monoclonal antibodies obtained as described above are of the class IgG, IgM, or IgA, and commonly react with many experimental Aspergillus strains and clinical isolants. For diagnosis and therapy, the IgG class is preferable.

Next, the present invention is explained in detail by Examples.

### Example 1

(1) Culturing of Aspergillus

A piece of mycelia of A. fumigatus (IFO 4057) was taken from a slant culture and inoculated to 100 ml of a medium (0.5% yeast extract, 1% Polypepton and 2.0% glucose), and aelobically cultured at 30°C overnight with shaking. 100 $\mu$l of this cultured broth was inoculated to a 20 ml plate (the above-mentioned medium + 1.5% agar), and cultured at 30°C for two days. Then 100 ml of PBS containing 1% formaldehyde was added to this plate, and a reaction was carried out at room temperature for one hour. After separating the mycelia from the plate, centrifugation was carried out at 2000 xg for 10 minutes to precipitate the Aspergillus mycelia, which were then resuspended in 10 ml of phosphate-buffered saline (PBS) followed by centrifugation, to wash the cells. After this procedure was repeated once again, the mycelia were suspended in 40 ml of distilled water and disrupted by a Waring blendor for three minutes. This disruptant was washed by centrifugation, the precipitate was suspended in 10 ml of distilled water, and the suspension was frozen for storage. Other Aspergillus strains were treated by the same procedure as described above, to prepare antigenic materials.

(2) Enzyme-linked immunosorbent assay (ELISA)

The above-mentioned stock cell suspension was thawed, to 2 to 3 ml of the cell suspension was added 500 ml of pure water, and the diluted cell suspension was added to an ELISA plate (Falcon 3912 Microtest III flexible assay plate, 96 wells) at an amount of 0.03 ml/well. After heating the plate at 65°C for 5 hours to evaporate liquid, 0.05 ml of 0.1% glutalaldehyde was put into each well to carry out a reaction for 10 minutes, followed by removal of the supernatant. Next, 0.15 ml of PBS supplemented with 0.1% $NaN_3$ and 1% bovine serum albumin (BSA) was put into each well, and after allowing to stand at 37°C for one hour, the plate was stored at 4°C, to be used as an assay plate for an anti-Aspergillus antibody assay. Then, 60 μl of a hybridoma culture supernatant was added to this plate, which was allowed to stand at a room temperature for one hour. After three times washing with PBS containing 0.05% Tween, 60 μl of goat anti-human IgG or IgM antibody-alkaline phosphatase (diluted 2000-fold) was added to this plate, and a reaction was carried out at a room temperature for one hour. After an additional three-times washing, 100 μl of a solution prepared by dissolving P-nitrophenylphosphate in 1 M diethanolamine + 1 mM $MgCl_2$ (pH 9.8) at a ratio of 0.6 mg/ml was added to the plate, and after 30 to 60 minutes, the absorbance at 405 nm was measured.

To screen hybridomas and EB virus-transformed lymphoblast cells, A. fumigatus (IFO 4057) was used.

(3) Human tonsil lymphocyte

Human tonsils removed by surgery were disrupted with forceps and scissors in ice-cold RPMI 1640 to obtain cells. The cells were twice washed by dispersing the cells in ice-cold RPMI 1640 and precipitating them by centrifugation, and finally dispersed in RPMI 1640 containing 20% FCS, 10% dimethylsulfoxide and 80 μg/ml gentamycin to a concentration of about $5 \times 10^7$ cells/ml. One ml each of the dispersion was put into vials and stored in liquid nitrogen. Upon use, the frozen cell dispersion was thawed and put on Ficoll-Paque (Pharmacia), followed by centrifugation at 2000 rpm for 30 minutes to gather monocytes in an upper layer of the Ficoll Paque, and the monocytes were used as lymphocytes.

(4) Comparison in vitro of anti-Aspergillus
    antibody productivity

Lymphocytes prepared from tonsils removed from 34 patients were dispersed in culture medium A (RPMI 1640, 10% FCS, 20 mM HEPES, 2 mM glutamine, 1 mM pyruvate, 0.02 mg/ml serine and 80 $\mu$g/ml gentamycin) to a concentration of 2 x $10^6$ cells/ml, and after the addition of 20 $\mu$g/ml PWM, culturing was carried out for 6 days. The culture supernatant was obtained and assayed for anti-<u>Aspergillus</u> IgG according to ELISA described in paragraph (2). Table 1 shows an amount of anti-<u>Aspergillus</u> IgG antibody produced in vitro by each tonsillar lymphocyte. The numerical values are the absorbance at 405 nm. From this result, it was found that the tonsils producing IgG antibody in a large amount were 6, 11, 15, 21 and 33.

EP 0 352 328 A1

<u>Table 1</u>

| Tonsil No. | | 1 | 2 | 3 | 4 | 5 | 6 | 7 | 8 | 9 | 10 | 11 | 12 |
|---|---|---|---|---|---|---|---|---|---|---|---|---|---|
| Antibody production | PWM | 2.7 | 1.7 | 1.0 | 1.0 | 1.7 | 8.7 | 2.0 | 1.0 | 0.3 | 2.3 | 4.7 | 1.3 |
| | BCGF | 2.3 | 1.0 | 1.0 | 1.0 | 3.0 | 8.0 | 2.0 | 1.0 | 1.0 | 1.0 | 3.3 | 1.0 |

| Tonsil No. | | 13 | 14 | 15 | 16 | 17 | 18 | 19 | 20 | 21 | 22 | 23 | 24 |
|---|---|---|---|---|---|---|---|---|---|---|---|---|---|
| Antibody production | PWM | 2.3 | 1.3 | 4.3 | 1.0 | 1.0 | 0.0 | 0.0 | 1.0 | 3.3 | 0.3 | 0.7 | 0.7 |
| | BCGF | 2.0 | 1.7 | 3.0 | 1.3 | 2.7 | 0.0 | 0.3 | 1.0 | 3.0 | 1.0 | 1.0 | 0.7 |

| Tonsil No. | | 25 | 26 | 27 | 28 | 29 | 30 | 31 | 32 | 33 | 34 |
|---|---|---|---|---|---|---|---|---|---|---|---|
| Antibody production | PWM | 0.7 | 0.0 | 2.7 | 2.0 | 0.0 | 0.0 | 0.3 | 0.7 | 6.0 | 0.0 |
| | BCGF | 0.0 | 0.7 | 1.3 | 2.3 | 0.0 | 0.0 | 0.3 | 0.3 | 6.3 | 0.0 |

(5) Cell fusion

Lymphocytes obtained from the tonsils No. 11, 21, and 33 in Table 1 were dispersed in a culture medium A at a density of $2 \times 10^6$ cells/ml, 1/10 volume of BCGF was added to the dispersion, and culturing was carried out in 5% $CO_2$ at 37°C. The lymphocytes and mouse myeloma cells were subjected to cell fusion as follows. Prior to cell fusion, mouse myeloma P3x63 Ag8U1 cells were cultured in a culture medium (the culture medium A but excluding HEPES). $1 \times 10^7$ myeloma cells and $5 \times 10^6$ PWM-stimulated lymphocytes were mixed, washed with a serum-free RPMI 1640, and finally centrifuged at 1500 rpm for 5 minutes to discard the supernatant. The remaining cell pellet was gentlly disrupted by patting the test tube containing the pellet. To the test tube was added 1.0 ml polyethylene glycol solution (5.75 ml of RPMI 1640, 3.5 ml of polyethylene glycol 1000 and 0.75 ml of dimethylsulfoxide) (abbreviated as PEG solution), and the cells were gently suspended. After one minute, 1.0 ml PRMI 1640 was added to the cell suspension, followed by additions of 2.0 ml RPMI after an additional one minute, 4.0 ml HAT medium (RPMI 1640, 10% fetal calf serum, 80 μg/ml gentamycin, 95 μM hypoxanthine, 0.4 μM aminopterin and 1.6 μM thymidine) after an additional two minutes, and 8.0 ml HAT medium after an additional two minutes in this order. Finally, a HAT medium was added to provide a 125 ml cell suspension. The suspension was distributed to 5 culture plates (96 wells), and cultured at 37°C in 5% $CO_2$-containing air. At one-week intervals, a half of the culture medium was replaced with fresh HT medium (a medium the same as the HAT medium obtained by excluding A) to obtain hybridomas.

The hybridomas were screened for anti-Aspergillus IgG productivity by ELISA, and a part of the result of the ELISA is set forth in Table 2. It is seen that hybridomas (clones) producing an anti-<u>Aspergillus</u>
IgG antibody were very efficiently developed.

<u>Table 2</u>

| Cell fusion plate[(*)] | Value of ELISA (relative value of absorbance at A405 nm) | Number of clones |
|---|---|---|
| Plate No. 1 | > 10 | 1 |
| | 9 to 5 | 2 |
| | 4 to 2 | 9 |
| Plate No. 2 | > 10 | 0 |
| | 9 to 5 | 2 |
| | 4 to 2 | 8 |
| Plate No. 3 | > 10 | 0 |
| | 9 to 5 | 0 |
| | 4 to 2 | 4 |
| Plate No. 4 | > 10 | 0 |
| | 9 to 5 | 2 |
| | 4 to 2 | 12 |

(*) Each plate had 96 wells.

(6) Cloning of hybridomas

Cloning was carried out by a limiting dilution
technique. Namely, from anti-<u>Aspergillus</u> antibody
positive wells, cells were obtained, counted, and
distributed to wells of a plate at a ratio of one
cell/well or 10 cells/well, using a culture medium B
(RPMI 1640, 10% FCS, 2 mM glutamine, 1 mM pyruvate,
0.02 mg/ml serine and 80 μg/ml gentamycin). After two
weeks, the cells were sufficiently grown, and thus the
supernatants were assayed to determine the presence of
an anti-<u>Aspergillus</u> monoclonal antibody, by ELISA, to
select hybridomas capable of producing an
anti-Aspergillus monoclonal antibody.

In this manner hybridomas AAE6A11, PLR20B2,
PN04A2, PLT3H9 and PNM4G4 were established, and these hybridomas now stably continue to produce human monoclonal antibody.

(7) Specificity of human anti-<u>Aspergillus</u> monoclonal antibodies

It was found, by ELISA, that the human monoclonal antibodies PLR20B2, PN04A2, PLT3H9 and PNM4G4 are IgM antibody, and AAE6A11 is an IgG antibody, and thus their reactivity to various kinds of <u>A. fumigatus</u> strains were studied by ELISA. The results are shown in Table 3.

<u>Table 3</u>

| Human Monoclonal Antibody | <u>A. fumigatus</u> (*2) | | | | | | | | | | | Control | | |
|---|---|---|---|---|---|---|---|---|---|---|---|---|---|---|
| | 1 | 2 | 3 | 4 | 5 | 6 | 7 | 8 | 9 | 10 | 11 | 12 | 13 | 14 |
| AAE6A11 | (*1) 5 | 4 | 4 | 2 | 4 | 4 | 1 | 1 | 1 | 1 | 1 | 2 | 0 | 0 |
| PLR20B2 | 1 | 4 | 8 | 3 | 9 | 8 | 4 | 1 | 2 | 1 | 1 | 2 | 0 | 0 |
| PN04A2 | 1 | 5 | 6 | 3 | 8 | 5 | 2 | 1 | 2 | 1 | 1 | 4 | 0 | 0 |
| PLT3H9 | 2 | 5 | 8 | 5 | 9 | 8 | 5 | 2 | 3 | 3 | 3 | 4 | 0 | 1 |
| PNM4G4 | 6 | 8 | 3 | 3 | 5 | 5 | 5 | 5 | 5 | 4 | 9 | 7 | 0 | 0 |

(*1) Values represent result of ELISA

(*2) Strains of <u>A. fumigatus</u> are as follows.

| | | |
|---|---|---|
| 1. | <u>A. fumigatus</u> | IFO 4057 |
| 2. | " | " 4399 |
| 3. | " | " 4400 |
| 4. | " | " 5840 |
| 5. | " | IAM 2046 |
| 6. | " | " 2176 |
| 7. | " | " 2530 |
| 8. | " | " 3006 |
| 9. | " | AHU 7323 |
| 10. | " | " 7324 |

| 11. | A. fumigatus | AHU 7337 |
| 12. | " | " 7387 |
| 13. | Candida albicans | J 1012 |
| 14. | No addition of cells | |

(8)  Purification of human monoclonal antibody

Hybridoma AAE6A11 cells were dispersed in the medium B and cultured while maintaining a cell concentration of between $1 \times 10^5$ cells/ml and $10 \times 10^5$ cells/ml.  Then 740 ml of a resulting culture supernatant was applied to a protein A-Sepharose column (size 1.7 cm x 3.6 cm), and after washing the column with PBS and water, a human monoclonal antibody was eluted with a diluted hydrochloric acid solution (pH 2.5).  The elute was immediately neutralized by adding a one tenth volume of 10-fold concentrated PBS. Accordingly, about 4.6 mg of protein was obtained.  It was confirmed by electrophoresis that this protein is a purified antibody (molecular weight about 160,000); and by ELISA that it is an antibody to Aspergillus.

The above-mentioned hybridoma AAE6A11 was designated as AAE6A11(Asp) and deposited with the Fermentation Research Institute, Agency of Industry and Technology, 1-3 Higashi 1-chome Tsukuba-shi, Ibaraki-ken Japan, as an international deposition under the Budapest Treaty, as FERM BP-2075 on September 27, 1988.

Example 2

(1)  Transformation by EB virus

Human tonsil lymphocytes were obtained as described in Example 1, paragraph (3), and dispersed in the medium B to $1 \times 10^6$ cells/ml as described in Example 1, paragraph (6).  Then, 10 ml of the dispersion and 2 ml of B95-8 cell culture supernatant (containing EB virus) were mixed and incubated at 37°C for one hour. The mixture was then centrifuged at 1500 rpm for 5 minutes to obtain a cell pellet, to which was added 100 ml of medium B containing 20% FCS.  This lymphocyte dispersion was distributed to 10 plain-bottomed 96-well plates.  Note, peritoneal exudate cells were previously added to the plate as feeder cells.

Cells distributed to these plates were cultured at 37°C in humid air containing 5% $CO_2$ , and at intervals of 7 to 10 days, a half of the culture medium was replaced with fresh medium.  After about 4 weeks, the EB virus-transformed lymphoblast cells developed colonies in a about 90% of the wells.  Supernatants from these wells were obtained and screened by ELISA for the presence of a human monoclonal antibody (both IgG and IgM) to Aspergillus.  The results are shown in Table 4. As seen from Table 4, several clones exhibiting a high value were formed.

Table 4

| ELISA value (relative value of absorbance A 405 nm | Number of clone[*] |
|---|---|
| > 10 | 5 |
| 9 to 5 | 41 |
| 4 to 2 | 173 |

(*)  Number of antibody-producing clones developed from 8 plates i.e., 768 wells.

(2)  Cloning of EB virus-transformed cell

The limiting dilution technique described in Example 1, paragraph (6) was used, except that cells were distributed to wells at a ratio of 5 cells/well to 50 cells/well.  The resulting supernatants were assayed by ELISA for the presence of an anti-Aspergillus antibody, and 16 kinds of parent cells were finally cloned to obtain cell clone AAG5B1 persistently producing an IgM antibody to Aspergillus.

- 19 -

(3) Specificity of anti-Aspergillus monoclonal antibody AAG5B1

The specificity was tested in the same manner as described in Example 1, paragraph (7), and the results are shown in Table 5. It was found that, although the AAG5B1 reacts with a wide spectrum of the genus Aspergillus, it does not react with Candida.

Table 5

| Antigen (*) | 1 | 2 | 3 | 4 | 5 | 6 | 7 |
|---|---|---|---|---|---|---|---|
| ELISA value | 8 | >10 | 5 | 6 | 7 | 9 | 5 |

| Antigen (*) | 8 | 9 | 10 | 11 | 12 | 13 | 14 |
|---|---|---|---|---|---|---|---|
| ELISA value | 5 | 7 | 5 | >10 | >10 | 0 | 0 |

(*) See Table 3.

Reference to microorganism deposited under Pule 13-2
Depository Authority: Ministry of Trade and Industry, Fermentation Research Institute, Agency of Industry and Technology
Address: 1-3 Higashi 1-chome Tsukuba-shi, Ibaraki-ken Japan
Receipt number and date of deposition
  1. FERM BP-2075   September 27, 1988

INTERNATIONAL FORM

BUDAPEST TREATY ON THE INTERNATIONAL
RECOGNITION OF THE DEPOSIT OF
MICROORGANISMS FOR THE PURPOSES OF
DEPOSITOR               PATENT PROCEDURE
TEIJIN LIMITED
Representative          RECEIPT IN THE CASE OF AN ORIGINAL
SASHIRO OKAMOTO         DEPOSIT
ADDRESS                 issued pursuant to rule 7.1 by the
11 Minamihoncho         INTERNATIONAL DEPOSITARY AUTHORITY
1-chome,                identified at the bottom of this
Higashi-ku              page.
Osaka-shi 541

I.    IDENTIFICATION OF THE MICROORGANISM

Identification reference given by the
DEPOSITOR:                   (Deposition Number)
     AAE6A11 (AsP)          FERM BP-2075

II.   SCIENTIFIC DESCRIPTION AND/OR TAXONOMIC
      DESIGNATION

The microorganism identified under I above was
accompanied by:

       a scientific description
  X   a proposed taxonomic designation
(Mark with a cross where applicable)

III.   RECEIPT AND ACCEPTANCE

This International Depositary Authority accepts the
micro-organism identified under I above, which was
received by it on organism identified under I above,
which was received by it on September 27, 1988 (Date of
the original deposit)[1].

IV.   INTERNATIONAL DEPOSITARY AUTHORITY

Fermentation Research Institute

Agency of Industrial Science and Technology
Director General  Tomoo Suzuki

1-3, Higashi 1-chome, Tsukuba-shi,
Ibaraki-ken, 305, Japan

Spetember 28, 1988

## CLAIMS

1. A human monoclonal antibody to Aspergillus.

2. A human monoclonal antibody according to claim 1, produced by a hybridoma formed from cell fusion between human lymphocyte and an established lymphoid cell, and/or a cell line derived therefrom.

3. A human monoclonal antibody according to claim 2, wherein the established lymphoid cell is a mouse myeloma cell.

4. A hybridoma producing a human monoclonal antibody to Aspergillus, formed from cell fusion between human lymphocyte and an established lymphoid cell, and a cell line derived from the hybridoma.

5. A process for production of a human monoclonal antibody to Aspergillus, characterized by culturing hybridoma producing a human monoclonal antibody to Aspergillus, formed from cell fusion between human lymphocyte and an established lymphoid cell, or a cell line derived from the hybridoma, and recovering the human monoclonal antibody from the culture.

6. A human monoclonal antibody according to claim 1, produced by a transformed cell obtained by transforming human lymphocyte with an EB virus, and a cell line derived from the transformed cell.

7. A transformed cell producing a human monoclonal antibody to Aspergillus, obtained by transforming human lymphocyte with EB virus, and a cell line derived from the transformed cell.

8. A process for production of a human monoclonal antibody to Aspergillus, characterized by culturing a transformed cell producing a human monoclonal antibody to Aspergillus, obtained by transforming a human lymphocyte with EB virus, and/or a cell line derived from the transformed cell, and recovering the human monoclonal antibody from the culture.

# INTERNATIONAL SEARCH REPORT

International Application No    PCT/JP88/01053

## I. CLASSIFICATION OF SUBJECT MATTER (if several classification symbols apply, indicate all) ⁶

According to International Patent Classification (IPC) or to both National Classification and IPC

Int.Cl⁴    C07K15/04, C12N5/00, 15/00, C12P21/00//A61K39/395,
G01N33/577 (C12P21/00, C12R1:91)

## II. FIELDS SEARCHED

Minimum Documentation Searched ⁷

| Classification System | Classification Symbols |
| --- | --- |
| IPC | C07K15/04, C12N5/00, 15/00, C12P21/00, A61K39/395, G01N33/577 |

Documentation Searched other than Minimum Documentation
to the Extent that such Documents are Included in the Fields Searched ⁸

## III. DOCUMENTS CONSIDERED TO BE RELEVANT ⁹

| Category * | Citation of Document, ¹¹ with indication, where appropriate, of the relevant passages ¹² | Relevant to Claim No. ¹³ |
| --- | --- | --- |
| X | JP, A, 59-187795 (Kureha Chemical Industry Co., Ltd.) 24 October 1984 (24. 10. 84) & GB, A, 2138445 & FR, A, 2543970 | 1-8 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
| --- | --- |
| December 26, 1988 (26. 12. 88) | January 17, 1989 (17. 01. 89) |

| International Searching Authority | Signature of Authorized Officer |
| --- | --- |
| Japanese Patent Office | |

Form PCT/ISA/210 (second sheet) (January 1985)